(19) Europäisches Patentamt
European Patent Office
Office européen des brevets

(11) Publication number: 0 040 053
B1

(12) EUROPEAN PATENT SPECIFICATION

(45) Date of publication of patent specification: 21.11.84

(51) Int. Cl.$^3$: A 61 N 1/42

(21) Application number: 81302023.7

(22) Date of filing: 07.05.81

# (54) Magnetic treatment apparatus.

(30) Priority: 08.05.80 JP 59976/80
13.05.80 JP 63717/80
30.05.80 JP 71484/80
28.07.80 JPU 105672/80

(43) Date of publication of application:
18.11.81 Bulletin 81/46

(45) Publication of the grant of the patent:
21.11.84 Bulletin 84/47

(84) Designated Contracting States:
DE FR GB IT

(56) References cited:
BE-A- 732 425
DE-A-2 506 227
DE-A-2 827 990
FR-A-2 210 420

(73) Proprietor: INOUE-JAPAX RESEARCH INCORPORATED
5289 Aza Michimasa Nagatsudamachi Midoriku
Yokohamashi Kanagawaken (JP)

(72) Inventor: Inoue, Kiyoshi
3-16-8 Kamiyoga
Setagayaku Tokyo (JP)

(74) Representative: Saunders, Harry
SAUNDERS & DOLLEYMORE 2 Norfolk Road
Rickmansworth Hertfordshire WD3 1JH (GB)

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

EP 0 040 053 B1

Courier Press, Leamington Spa, England.

## Description

The present invention relates in general to a magnetic medical treatment apparatus. More particularly, it relates to an improved apparatus for magnetic treatment to relieve pain and cure disease, also called "magnetic acupuncturing".

It has been widely recognized that magnetism is effective to relieve a stiff neck and to cure other body troubles and malfunctions such as hypertension. Thus, a variety of adhesive plasters and other forms of magnetic curing aids and devices have been produced, and been used by individuals.

On the effect of curing by magnetism, the theory widely accepted is that an electric current caused in a flow of blood under a magnetic field gives rise to a stimulus or impetus to an autonomic nervous system, thereby normalizing its functions in an individual.

Magnetic acupuncturing or treatment devices which have hitherto been proposed in the art make common use of a fixed permanent magnet or electromagnet (i.e. energized by a direct current) so that a static magnetic field may be provided and applied to a body surface, in particular to an affected zone thereon or to one or more of what are called "tsubo" or key points which have been well established according to the Chinese or oriental medicine to be distributed over the surface of a human body.

German Patent Specification No 2 827 990 discloses a massage device which not only subjects the area to be treated to a mechanical effect but also to a magnetic effect. The magnetic effect is achieved by the displacement of a magnet to increase and decrease the level of magnetic field to which the area under treatment is subject.

The present invention seeks to provide (a) a new and useful improved magnetic medical treatment apparatus which is relatively simple and inexpensive and yet capable of achieving increased curing effects; (b) a compact magnetic medical treatment device which can be used adjustably for a variety of curing purposes; and (c) a versatile magnetic medical treatment apparatus which can be used adjustably by a practitioner for a patient or by and for different individuals for a variety of curing purposes. Accordingly the present invention is characterised in that the at least one magnet is so aligned that the direction of the magnetic field which causes the magnetic flux penetrating a fixed point of said preselected zone dynamically alternates with a frequency which is an integral multiple of the rotary frequency.

Other objectives will become apparent as the following description proceeds.

These and other features and advantages of the present invention will become more readily apparent from the following description of certain embodiments thereof as taken with reference to the accompanying drawings in which:

Fig. 1 is an elevational view essentially in section, diagrammatically illustrating a magnetic medical treatment apparatus embodying the present invention using a rotating magnet;

Fig. 2 is an elevational sectional view diagrammatically illustrating a further embodiment of the invention having a rotary disc carrying a plurality of permanent magnets detachably and replaceably secured thereon;

Fig. 3 is a sectional diagrammatic view taken along the line X—X in Fig. 2;

Fig. 4 is an elevational view, essentially in section, diagrammatically illustrating a rotary arrangement which carries a plurality of permanent magnets on a rotating disc, the magnets being also individually rotatable on their own axes;

Fig. 5 is a plan view essentially in section taken along the line XII—XII in Fig. 4;

Fig. 6 is an elevational view, partly in section and partly schematic, diagrammatically illustrating a further rotary apparatus embodying the present invention; and

Fig. 7 is a plan view essentially in section taken along the line XIV—XIV in Fig. 6.

Referring first to Fig. 1, a magnetic medical treatment device shown includes a plastic casing 1 in the form of a cylindrical cup and having a projection 1*a* for engagement with a preselected zone, e.g. an affected area, or a preselected "tsubo" or key point, on the body surface of a patient. In the casing 1, a rotary shaft 2 having a permanent magnet member 3 securely mounted thereon is journalled in a bearing 4 fitted immediately above the projection 1*a*. The permanent magnet member 3 is formed with a pair of legs 3*a* and 3*b* whose ends are positioned symmetrically about the shaft 2 and serve as N and S poles, respectively, as shown, or with two or more such pairs arranged in the form of spider legs. The two or more (say, six) poled magnetic legs or projections 3*a* and 3*b* set up a magnetic field of desired strength (e.g. 300 to 10000 Gauss) in the region of the zone on the body surface in engagement with the projection 1*a*. The shaft 2 carries a wheel 5 secured thereon which is in pressure engagement with a rubber wheel 6 securely carried on a drive shaft 7 of an electric motor 8 of miniature type. The latter has input leads 9 energized by a commercial AC supply, or a battery (not shown) which may be accommodated in the casing 1. The motor 8 is fitted in a plastic support 10 secured to an inner wall portion of the casing 1. The latter is threadedly fitted with a plastic or rubber cap 11.

In manipulation, the casing 1 is manually supported to bring the projection 1*a* into engagement with an affected zone, or a preselected "tsubo" or key point on the body surface of an individual to press the zone or point under an appropriate pressure and the

motor 8 may be energized to rotate the drive shaft 7. The rotation is transmitted via the wheels 6 and 5 to the shaft 2 to rotate the permanent magnet member 3. This causes the magnetic field set up in the body surface by the poles 3*a* and 3*b* to be dynamically fluctuated (i.e. by relative movement of the poles and casing). The result is that the zone or point is placed under the synergistic actions by the pressure stimulus and the dynamic field fluctuation of the device to yield an enhanced curing effect.

The device of Fig. 1 is shown further including means to achieve the rotation of the shaft 2 with other than the motor 8. The shaft 2 is thus drivably coupled with a unidirectional clutch 12 fitted in the support 10 and having a shaft 13 rotatably received in a bearing 14. The shaft 13 is secured to a bevel gear 15 in mesh with a bevel gear 16 securely carried on a shaft 17. A spur gear 18 is also securely carried on the shaft 17 and in mesh with a gear section 19*a* of a swingable lever 19 which is pivoted on a shaft 20. A fixed lever 21 has a pair of arms 21*a* carrying the shaft 20, and is fixed to an inner wall portion of the casing 1 with rivets 22 and 23. The swingable lever 19 has a lever section 19*b* biassed away from the lever portion 21*b* of the fixed lever 21 by a spring 24, which urges the lever 19 about the shaft 20 in the clockwise direction.

In manipulation, the lever portions 19*b* and 21*b* of the swingable and fixed levers 19 and 21 are gripped so as to be brought together against the spring pressure. This causes a rotation of the gear section 19*a* of the lever 19 about the shaft 20 in the counterclockwise direction. The rotation is transmitted via the spur gear 18, shaft 17, bevel gears 16 and 15 to the drive shaft 13 and, in turn, to the drive shaft 2 via the clutch 12 to rotate the magnet member 3. The lever 19 may then be released to allow its gear section 19*a* to rotate in the clockwise direction under the spring pressure and, in turn, to cause the spur gear 18, shaft 17 and bevel gears 16 and 15 to rotate back to their respective original angular positions. Since the unidirectional clutch 12 is at this time incapable of coupling the shaft 13 to the shaft 2, this reversed rotation is not transmitted to the shaft 2 and the magnet member 3 simply keeps rotating by virtue of its inertia. Additional rotary impulses may be applied to the magnet member 3 by repetitively gripping the levers 19*b* and 21*b*.

Figs. 2 and 3 show another form of the apparatus embodying the invention in which the dynamically fluctuated magnetic field is applied to a preselected zone on the body surface of a patient or individual. The apparatus makes use of a rotary disc 561 which carries a plurality of permanent magnets 503*a*, 503*b*, 503*c*, 503*d*, 503*e* and 503*f*. The disc 561 is secured on a shaft 502 by means of nuts 562 and has a plurality of openings 563*a*, 563*b*, 563*c*, 563*d*, 563*e* and 563*f* into which the magnets 503*a*, 503b, 503*c*, 503*d*, 503*e* and 503*f* are inserted and detachably fixed individually by means of a spring projection 564 formed on a wall portion of each opening.

The shaft 502 is journalled in a bearing 504 fitted in a support disc 510 secured to the inner wall of a casing 501 and has a gear 565 in mesh with a gear 566 rotatable by an electric motor 508 of miniature type mounted on the support disc 510. The motor 508 is energized by batteries 567*a* and 567*b* securely seated on the floor of the casing 501 via a drive control circuit unit 568 (including a driver circuit for the motor and switches) also securely seated on the floor of the casing 501. A cap 511 is threadedly fitted with the casing 501 to seal the foregoing components of the apparatus therein.

The permanent magnets 503*a* through 503*f* are shown arranged on the rotary disc 561 radially symmetrically about the shaft 502 and are revolved immediately below the cap 511 as the motor 508 is driven to rotate the drive shaft 502. The casing 501 may be manually carried to bring a preselected zone of the body surface of an individual in contact with the cap 511 in a region thereof below which the magnets 502*a* through 503*f* pass as they are revolved. In the example shown, the magnets 503*a*, 503*b* and 503*c* are positioned to direct their N poles upwards and the magnets 503*d*, 503*e* and 503*f* are positioned to direct their S poles upwards. As the disc 561 rotates, the preselected surface zone is consequently placed under a magnetic field whose polarity is successively switched in the sequence of

N—N—N—S—S—S—N—N—N— . . .

and hence under a predetermined format of the dynamically fluctuated magnetic field. As a result of the fact that the field fluctuates both in its strength and polarity, the current electromagnetically induced in the blood flow fluctuates both in its magnitude and direction in a given format. An optimum format can therefore be chosen to give rise to an optimum curing effect. The rate of switching of the magnetic field can be altered as desired by acting on the drive control circuit unit 568 for the motor 508. It has generally been found that successive magnetic fields should occur at a rate of 1 to 100 Hz. Accordingly, in the example shown, i.e. with six magnets 503*a* through 503*f* mounted on the disc 561, the disc 561 should rotate at 1/6 to 20 rpm.

A desired polarity sequence of the dynamically fluctuated magnetic field can be set up by arranging the magnets 503*a* through 503*f* to direct their poles in a format corresponding to the desired sequence. For example, formats of

N—N—N—N—N—N— . . . . . ,

N—S—N—S—N—S— . . . . .

and

N—N—S—S—N—N—S— . . . . .

and practically any other format of polarity switching sequence can be achieved. In each case, each pole gradually approaches the selected zone and then goes away. Consequently, the strength of the magnetic field periodically varies in a pulsating form. It has been found that such form of variation in the field strength gives rise to a markedly better curing effect than the use of a fixed constant field strength. Each of the magnets 503*a* to 503*f* should have a magnetic strength in the range, say, between 300 and 3000 Oersteds.

In a modified embodiment shown in Figs. 4 and 5 of the rotary system, in which same references are used to designate same components as in Figs. 2 and 3, the rotary disc 561 has a plurality of stands 670*a*, 670*b*, 670*c* and 670*d* securely mounted thereon which have their respective shafts 671*a*, 671*b*, 671*c* and 671*d* driven by pulse motors 672*a*, 672*b*, 672*c* and 672*d*, respectively. The shafts 671*a*, 671*b*, 671*c* and 671*d* have permanent magnets 603*a*, 603*b*, 603*c* and 603*d* securely carried thereon, respectively at their respective centers. The pulse motors 672*a* through 672*d* are mounted on the rotary disc 561 and driven by a driver circuit (not shown) constructed in a unit mounted, for example, to depend from the rotary disc 561. The driver circuit is used to incrementally rotate the respective pulse motors 672*a* through 672*d* individually to rotate and position the magnets 603*a*, 603*b*, 603*c* and 603*d* to make them individually establish their magnetic fields of desired polarities. It is also possible to constitue the motors 672*a*, 672*b*, 672*c* and 672*d* each with a DC motor and to keep them also rotating constantly as the supporting disc 561 is rotated in operation of the apparatus. Then by permitting the DC motors to rotate at different speeds and the latter to be adjustably varied it is possible to enable a choice among quite a diverse range of possibilities of the field polarity sequence and strength variation in operation of the apparatus.

In a further modification shown in Figs. 6 and 7, in which same references are used to designate same components as in the embodiment of Figs. 2 and 3 and the embodiment of Figs. 4 and 5, the stands 670*a*, 670*b*, 670*c* and 670*d* for rotatably carrying the magnets 603*a*, 603*b*, 603*c* and 603*d* on their respective shafts 671*a*, 671*b*, 671*c* and 671*d* have leaf springs 673*a*, 673*b*, 673*c* and 673*d* secured thereto, respectively, to urge wheels 674*a*, 674*b*, 674*c* and 674*d*, each in the form of a circular truncated cone secured to the shaft 671*a*, 671*b*, 671*c*, 671*d*, to mate with a wheel 675, also in the form of a circular truncated cone but of a larger size secured to the cap 511 of the apparatus. The mating surfaces of the wheels 674*a* through 674*d* are composed of a high friction-coefficient material such as rubber. The mating surface of the wheel 675 should be formed with a fine rugged formation (e.g. knurled) to increase the friction with the wheels 674*a* through 674*d*. As the rotary disc 561 is rotated in the direction of the arrow, the wheels 674*a*, 674*b*, 674*c* and 674*d* are thus also rotated to rotate the magnets 603*a*, 603*b*, 603*c* and 603*d* supported respectively on their shafts 671*a*, 671*b*, 671*c* and 671*d* in the direction indicated by the respective arrows.

## Claims

1. A magnetic medical treatment apparatus comprising:

at least one magnet (3; Fig. 1: 603*a*; Fig. 4) having at its two opposite ends (3*a*,3*b*) a N-pole and a S-pole respectively for producing, in the proximity thereof, magnetic fluxes each of which is closed with said N- and S-poles;

support means (1,10; Fig. 1) for positioning said magnet (3:603*a*) in a predetermined proximity relationship with a preselected zone beneath the body surface of an individual such that at least a portion of said magnetic fluxes penetrates said zone; and

rotary drive means (5—8, 12—21; Fig. 1: 672*a*; Fig. 4) drivingly connected to said magnet (3:603*a*) for rotating said magnet (3:603*a*) about a rotary axis (2; Fig. 1:671*a*; Fig. 4) characterised in that the at least one magnet is so aligned that the direction of the magnetic field which causes the magnetic flux penetrating a fixed point of said preselected zone dynamically alternates with a frequency which is an integral multiple of the rotary frequency.

2. The apparatus defined in Claim 1, characterised in that said magnet (3:603*a*) is a permanent magnet, and said drive means includes an electric motor (8:672*a*) for rotating said magnet (3:603*a*) about said axis thereof.

3. The apparatus defined in Claim 1 or 2, characterised in that said magnet (603*a*) is one of a plurality of magnets (603*a*—*d*) disposed on a rotary disc member (561) and arranged so as to surround a rotary axis (502) thereof, and said apparatus further comprises a further rotary drive means (508,566,565) for rotating said rotary disc member (561) to revolve said plural magnets (603*a*—*d*) about the second-mentioned rotary axis (502) while each of said magnets (603*a*—*d*) is rotated about the first-mentioned rotary axis (671*a*—*d*).

4. The apparatus defined in Claim 1 or 2, characterised in that said support means (1,10) includes a housing (1,10,11) and said drive means includes manually operable reciprocation means (12—21) drivingly connected with

said magnet (3) for rotating said magnet (3) about said axis (2).

5. The apparatus defined in Claim 1, 2 or 4, characterised in that said support means (1,10) includes a projecting portion (1*a*) adapted to bear against said body surface above said preselected zone to establish paths for said penetrating magnetic fluxes.

**Revendications**

1. Appareil de traitement médical magnétique comprenant au moins un aimant (3; Fig. 1: 603a; Fig. 4) présentant, à ses deux extrémités opposées (3a,3b) respectivement des pôles Nord et Sud, afin de produire, à proximité de cet aimant, des flux magnétiques dont chacun se referme sur ses pôles Nord et Sud; un support (1,10: Fig. 1) pour mettre en position cet aimant (3: 603a) dans une relation de proximité prédéterminée par rapport à une zone préselectionnée située sous la surface du corps d'un individu de telle façon qu'au moins une partie de ces flux magnétiques pénètre dans ladite zone; et des moyens d'entraînement en rotation (5—8,12—21; Fig. 1: 672a; Fig. 4) accouplés à cet aimant (3: 603a) afin de faire tourner cet aimant (3: 603a) autour d'un axe de rotation (2; Fig. 1: 671a; Fig. 4), caractérisé en ce que cet aimant est aligné de telle façon que la direction du champ magnétique qui amène le flux magnétique à pénétrer en un point fixe de la dite zone préselectionnée, est modifiée alternativement et dynamiquement avec une fréquence qui est un multiple entier de la fréquence de rotation.

2. Appareil suivant la revendication 1 caractérisé en ce que l'aimant (3: 603a) est un aimant permanent et les moyens d'entraînement en rotation comportent un moteur électrique (8: 672a) afin de faire tourner cet aimant (3: 603a) autour de son axe.

3. Appareil suivant l'une quelconque des revendications 1 ou 2 caractérisé en ce que l'aimant (603a) est l'un d'une pluralité d'aimants (603a—d) disposés sur un disque rotatif (561) et agencés de manière à entourer un axe de rotation (502) de ce disque, et en ce qu'il comporte en outre des moyens d'entraînement en rotation additionnels (508,566,565) afin de faire tourner le disque rotatif (561), et ce de manière à entraîner la pluralité d'aimants (603a—d) autour du second axe de rotation mentionné (502), tandis que chacun des aimants (603a—d) est lui-même entraîné en rotation autour du premier axe de rotation mentionné (671a—d).

4. Appareil suivant l'une quelconque des revendications 1 ou 2 caractérisé en ce que le support (1,10) comporte un boîtier (1,10,11) et les moyens d'entrâinement comportent des moyens à mouvement alternatif actionnés manuellement (12—21) lesquels sont accouplés à l'aimant (3) afin de faire tourner cet aimant (3) autour de l'axe (2).

5. Appareil suivant l'une quelconque des revendications 1,2 ou 4 caractérisé en ce que le support (1,10) comporte une saillie (1a) adaptée de manière à porter contre la surface du corps au-dessus de la dite zone préselectionnée, afin d'établir des trajets pour les flux magnétiques pénétrants.

**Patentansprüche**

1. Gerät zur magnetischen, medizinischen Behandlung mit:

wenigstens einem Magnet (3; Fig. 1: 603a; Fig. 4), der an seinen entgegengesetzten Enden (3a, 3b) einen Nordpol bzw. einen Südpol zur Erzeugung von magnetischen Kraftflüssen in deren Nähe, von denen jeder mit den genannten Nord- bzw. Südpolen geschlossen ist;

einer Halteeinrichtung (1, 10: Fig. 1) zum Einstellen der Lage des Magneten (3: 603a) in einer vorbestimmten Nähebeziehung zu einer vorausgewählten Zone unter der Körperoberfläche eines Individuums, derart daß wenigstens ein Teil der genannten magnetischen Kraftflüsse diese Zone durchdringt;

und einer Drehantriebseinrichtung (5—8, 12—21; Fig. 1: 672a; Fig. 4), die mit dem genannten Magnet (3: 603a) zu dessen Rotation um eine Drehachse (2; Fig. 1: 671a; Fig. 4) drehverbunden ist,

dadurch gekennzeichnet, daß der wenigstens in Einzahl vorhandene Magnet so ausgerichtet ist, daß die Richtung des magnetischen Feldes, das den einen festen Punkt der vorausgewählten Zone durchdringenden magnetischen Kraftfluß erzeugt, dynamisch mit einer Frequenz wechselt, die ein ganzes Vielfaches der Drehfrequenz ist.

2. Gerät nach Anspruch 1, dadurch gekennzeichnet, daß der Magnet (3: 603a) ein Permanentmagnet ist und die Antriebseinrichtung einen Elektromotor (8: 672a) für die Drehung des Magneten (3: 603a) um dessen genannte Achse umfaßt.

3. Gerät nach Anspruch 1 oder 2, dadurch gekennzeichnet, daß der genannte Magnet (603a) einer in einer Mehrzahl von Magneten (603a—d) ist, die an einem drehbaren Scheibenglied (561) vorgesehen und so angeordnet sind, daß sie eine Drehachse (502) desselben umgeben, und daß das Gerät weiterhin eine weitere Drehantriebseinrichting (508, 566, 565) zur Drehung des drehbaren Scheibenglieds (561) umfaßt zum Drehen der mehreren Magnete (603a—d) um die zweiterwähnte Drehachse (502), während jeder dieser Magneten (603a—d) um die ersterwähnte Drehachse (671a—d) gedreht wird.

4. Gerät nach Anspruch 1 oder 2, dadurch gekennzeichnet, daß die genannte Halteeinrichtung (1,10) ein Gehäuse (1,10,11) umfaßt und daß die genannte Dreheinrichtung einemanuell betätigbare, hin- und hergehende Einrichtung (12—21) einschließt, die mit dem genannten Magnet (3) zwecks Drehung desselben um die genannte Achse (2) antriebsverbunden ist.

5. Gerät nach Anspruch 1, 2 oder 4, dadurch gekennzeichnet, daß die genannte Halteeinrichtung (1, 10) einen vorspringenden Teil (1a) zur Anlage an der Körperoberfläche über der ausgewählten Zone zur Bildung von Wegen für die hindurchdringenden magnetischen Kraftflüsse umfaßt.

FIG. 1

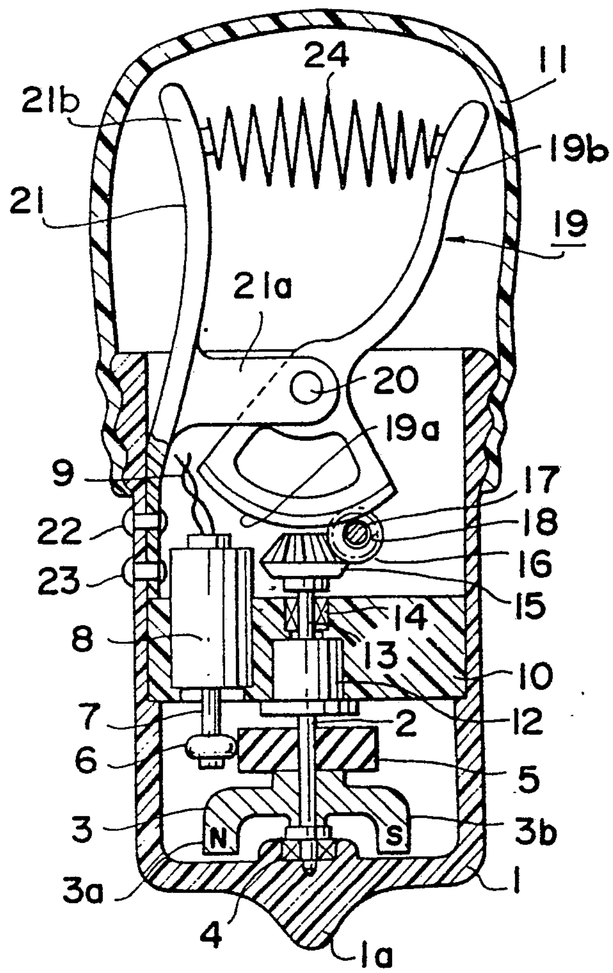
24
11
21b
19b
21
19
21a
20
19a
9
17
22
18
16
23
15
14
8
13
10
7
2
12
6
5
3
3b
N
S
3a
1
4
1a

FIG. 3

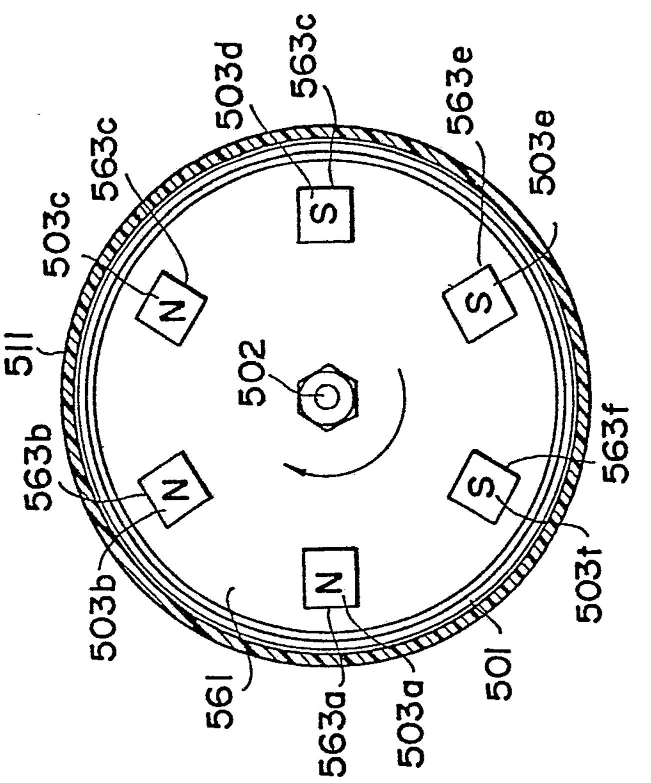

503d
563c
563e
503e
563c
503c
511
502
563b
563f
503b
503f
561
563a
503a
501
N
S


FIG. 2

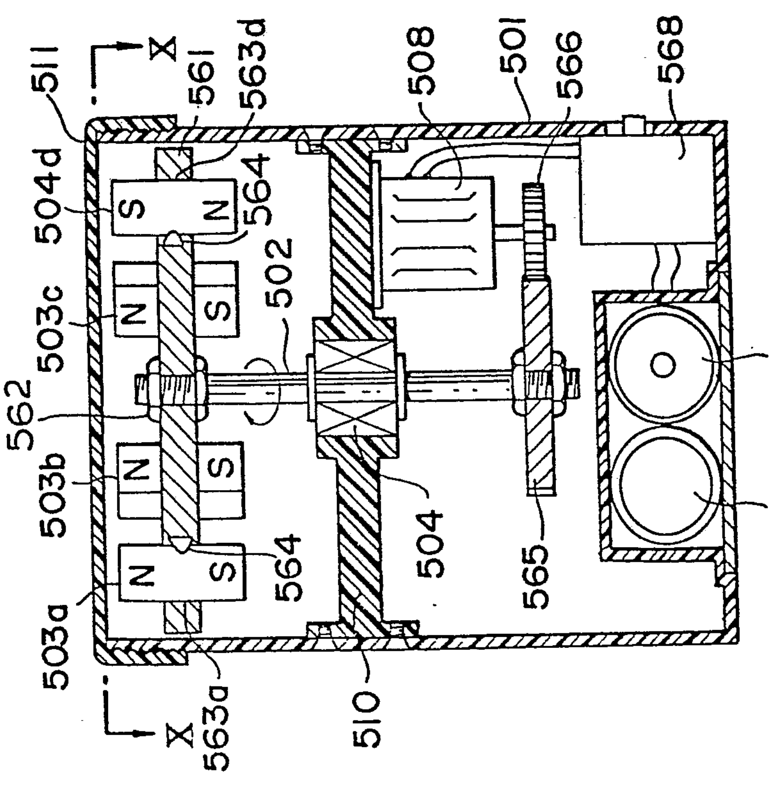

X
511
561
563d
508
501
566
568
504d
564
503c
502
562
503b
504
565
567b
567a
564
503a
510
563a
X
N
S

FIG. 4

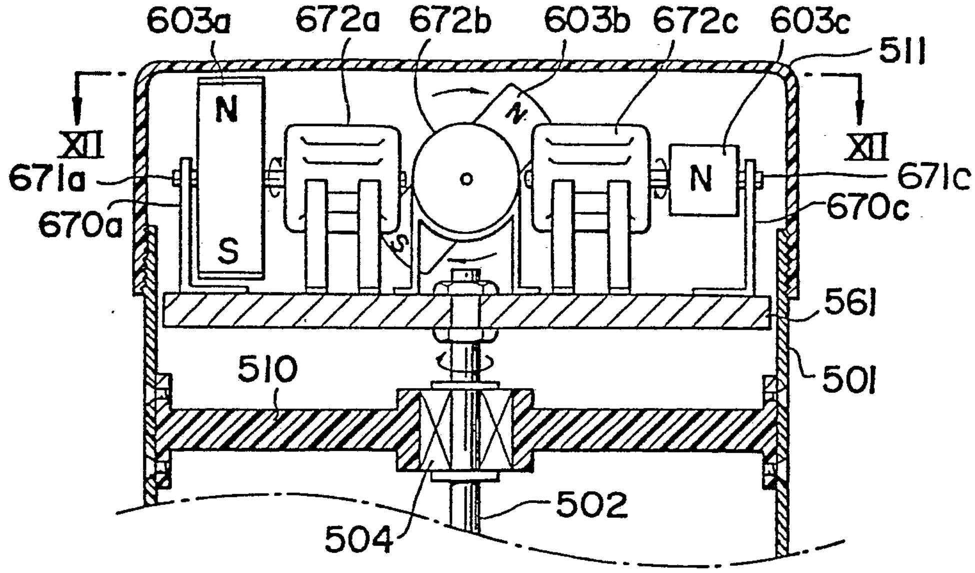
603a
672a
672b
603b
672c
603c
511
XII
XII
N
S
N
S
N
671a
670a
671c
670c
561
501
510
502
504

FIG. 5

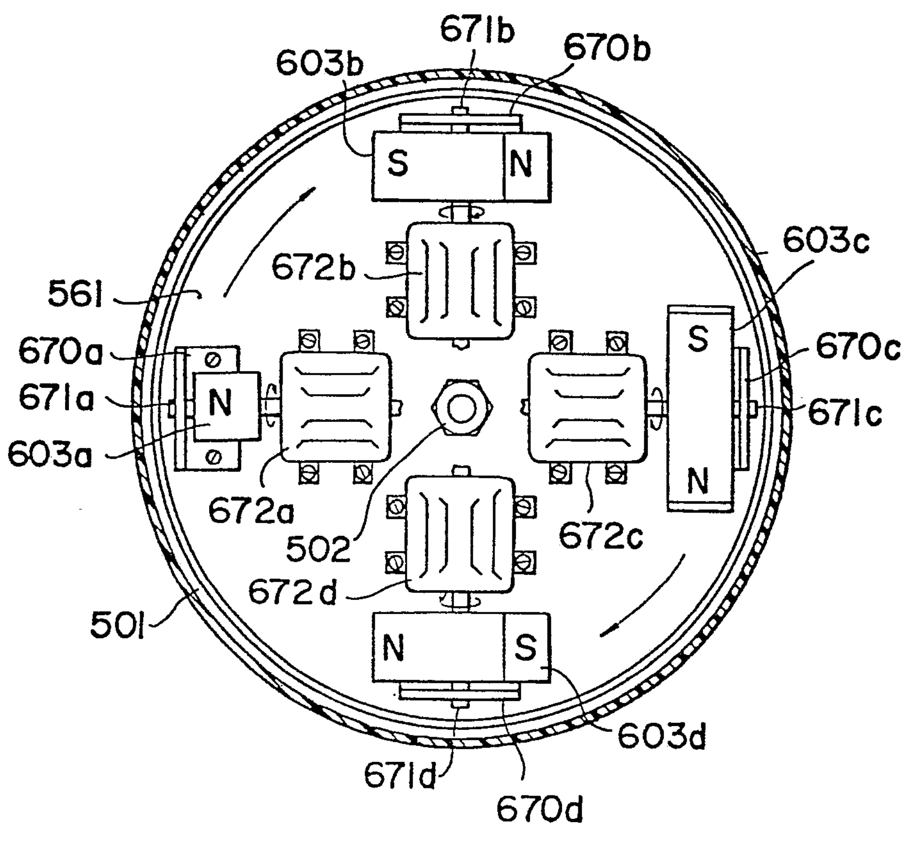
671b
670b
603b
S
N
672b
603c
561
S
670a
671a
603a
N
670c
671c
N
672a
502
672c
672d
501
N
S
603d
671d
670d

FIG. 6

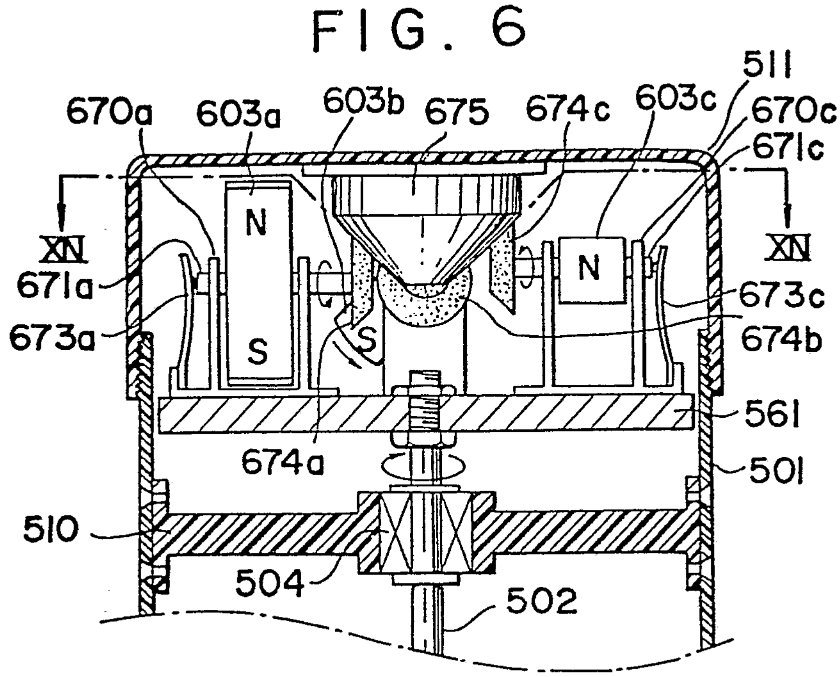
670a
603a
603b
675
674c
603c
511
670c
671c
XN
XN
671a
673a
N
S
S
N
673c
674b
561
674a
501
510
504
502

FIG. 7

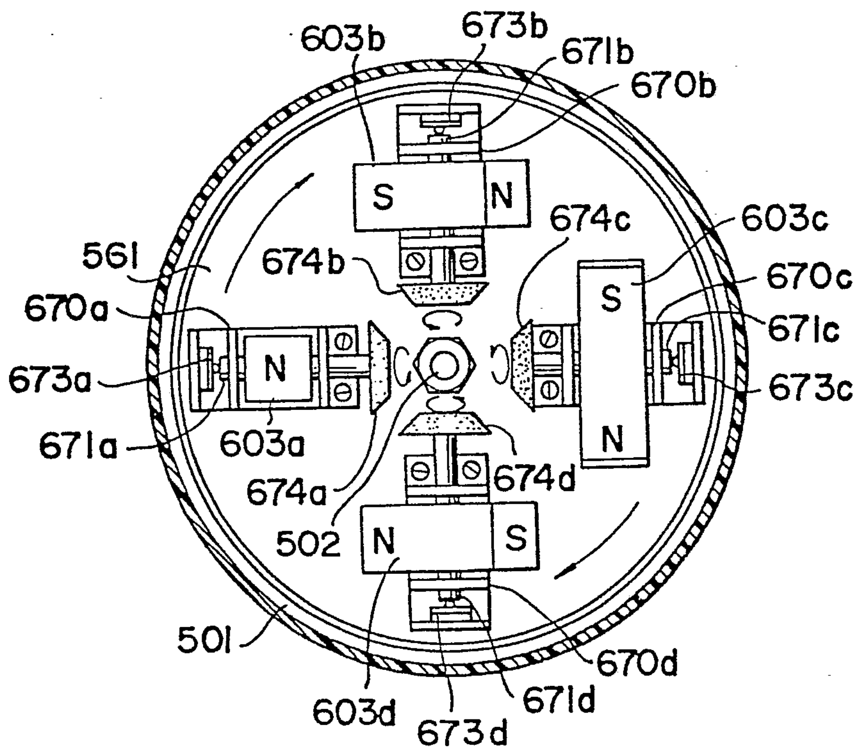
603b
673b
671b
670b
S
N
603c
674c
561
674b
670c
670a
S
671c
673a
N
673c
671a
603a
N
674d
674a
502
N
S
501
670d
603d
671d
673d